# EUROPEAN PATENT APPLICATION

(11) **EP 4 793 263 A1**
(43) Date of publication of application: **19.08.2026**
(21) Application number: 24877229.5
(22) Date of filing: 10.10.2024
(51) Int. Cl.: C07C 2/76, C07B 61/00, C07C 11/24

(54) **ACETYLENE PRODUCTION APPARATUS AND ACETYLENE PRODUCTION METHOD**

(30) Priority: 10.10.2023 JP 2023174919
(71) Applicant: NATIONAL UNIVERSITY CORPORATION KYOTO INSTITUTE OF TECHNOLOGY, Kyoto-shi, Kyoto 606-8585 (JP)
(72) Inventor: HIMURA, Haruhiko, Kyoto-shi, Kyoto 606-8585 (JP)
(74) Representative: Meissner Bolte Partnerschaft mbB
(86) International application number: PCT/JP2024/036256
(87) International publication number: WO 2025/079635

(57) **Abstract**

An acetylene production apparatus (1) includes: a chamber (101) that has a cylindrical shape, and is provided with, in at least a part of an upper wall (101b), an electromagnetic wave introduction portion (101d) for introducing a microwave along a cylinder axis (J1) direction; a first raw material gas supplier (111) that supplies a first raw material gas containing H₂ into the chamber (101); a second raw material gas supplier (121) that supplies a second raw material gas containing CH₄ into the chamber (101); and a plasma generator (103) that generates plasma (PLM) in the chamber (101) by introducing a microwave from the electromagnetic wave introduction portion (101d) into the chamber (101). Then, a shortest distance (L1) between the electromagnetic wave introduction portion (101d) and the inside of a side wall (101a) of the chamber (101) in a direction orthogonal to the cylinder axis (J1) direction of the chamber (101) is set longer than a vibration amplitude length of an electron in the plasma.

## Description

### Technical Field

The present invention relates to an acetylene production apparatus and an acetylene production method.

### Background Art

A method for producing acetylene by supplying gas containing at least one kind of hydrocarbon to plasma generated by a plasma source has been proposed (for example, see Patent Literature 1).

### Citation List

### Patent Literature

Patent Literature 1: Unexamined Japanese Patent Application Publication (Translation of PCT Application) JP 2015 - 516 958 A

### Summary of the Invention

### Technical Problem

In a method for producing acetylene using the plasma technique as described in Patent Literature 1, energy conservation is required while a yield of acetylene is increased.

The present invention has been made in view of the reasons described above, and has an object to provide an acetylene production apparatus and an acetylene production method that can increase a yield of acetylene while conserving energy.

### Solution to the Problem

An acetylene production apparatus according to the present invention includes:
a chamber that has a cylindrical shape, and is provided with, in at least a part of a wall blocking one end portion in a cylinder axis direction, an electromagnetic wave introduction portion for introducing an electromagnetic wave along the cylinder axis direction;
a first raw material gas supplier that supplies a first raw material gas containing H₂ into the chamber;
a second raw material gas supplier that supplies a second raw material gas containing CH₄ into the chamber; and
at least one plasma generator that generates plasma in the chamber by introducing an electromagnetic wave from the electromagnetic wave introduction portion into the chamber in a state where at least one of the first raw material gas and the second raw material gas is supplied in the chamber,
wherein a shortest distance between the electromagnetic wave introduction portion and the inside of a side wall of the chamber in a direction orthogonal to the cylinder axis direction of the chamber is set longer than a vibration amplitude length of an electron in the plasma.

An acetylene production apparatus according to another aspect of the present invention includes:
a chamber having a cylindrical shape;
a first raw material gas supplier that supplies a first raw material gas containing H₂ into the chamber;
a second raw material gas supplier that supplies a second raw material gas containing CH₄ into the chamber; and
at least one plasma generator that includes a coil disposed inside the chamber or outside the chamber and an alternating current source for applying an alternating current to the coil, and generates plasma in the chamber by applying an alternating current to the coil in a state where at least one of the first raw material gas and the second raw material gas is supplied in the chamber,
wherein a shortest distance between the coil and the inside of a side wall of the chamber in a direction orthogonal to a cylinder axis direction of the chamber is set longer than a vibration amplitude length of an electron in the plasma.

An acetylene production method according to another aspect of the present invention includes:
a first raw material gas supply step of supplying a first raw material gas containing H₂ into a chamber;
a first plasma processing step of generating ·H in advance in the chamber in a state where the first raw material gas is supplied in the chamber;
a second raw material gas supply step of supplying a second raw material gas containing CH₄ into the chamber after the first raw material gas supply step; and
a second plasma processing step of generating plasma in the chamber in a state where the first raw material gas and the second raw material gas are supplied in the chamber.

### Advantageous Effects of the Invention

According to the present invention, an acetylene generation reaction due to a collision of an electron in plasma with CH₄ is accelerated, and thus a yield of acetylene can be increased while conserving energy.

### Brief Description of the Drawings

FIG. 1 is a schematic diagram of an acetylene production apparatus according to Embodiment 1 of the present invention;
FIG. 2 is a schematic plan view illustrating a part of the acetylene production apparatus according to Embodiment 1;
FIG. 3A is a diagram illustrating a relationship between power and a vibration amplitude length of an electron when a frequency of an electromagnetic wave is 13.56 MHz;
FIG. 3B is a diagram illustrating a relationship between power and a vibration amplitude length of an electron when a frequency of an electromagnetic wave is 100 MHz;
FIG. 4 is a diagram illustrating a relationship between power and a vibration amplitude length of an electron when a frequency of an electromagnetic wave is 860 MHz;
FIG. 5 is a diagram illustrating a reaction occurring in a chamber;
FIG. 6 is a schematic diagram of an acetylene production apparatus according to Comparative Example;
FIG. 7 is a diagram illustrating a result of a component analysis by gas chromatography mass spectrometry for gas generated by the acetylene production apparatus according to Embodiment 1;
FIG. 8 is a schematic diagram of an acetylene production apparatus according to Embodiment 2;
FIG. 9 is a schematic plan view illustrating a part of the acetylene production apparatus according to Embodiment 2;
FIG. 10 is a schematic diagram of an acetylene production apparatus according to Modified Example; and
FIG. 11 is a schematic plan view illustrating a part of the acetylene production apparatus according to Modified Example.

### Description of Embodiments

### Embodiment 1

Hereinafter, an acetylene production apparatus according to embodiments of the present invention is described in detail with reference to drawings. An acetylene production apparatus according to the present embodiment includes: a chamber that has a cylindrical shape, and is provided with, in at least a part of a wall blocking one end portion in a cylinder axis direction, an electromagnetic wave introduction portion for introducing an electromagnetic wave along the cylinder axis direction; a first raw material gas supplier that supplies a first raw material gas containing hydrogen into the chamber; a second raw material gas supplier that supplies a second raw material gas containing CH₄ into the chamber; and at least one plasma generator that generates plasma in the chamber by introducing an electromagnetic wave from the electromagnetic wave introduction portion into the chamber in a state where the first raw material gas and the second raw material gas are supplied in the chamber. Then, a shortest distance between the electromagnetic wave introduction portion and the inside of a side wall of the chamber in a direction orthogonal to the cylinder axis direction of the chamber is set longer than a vibration amplitude length of an electron in the plasma.

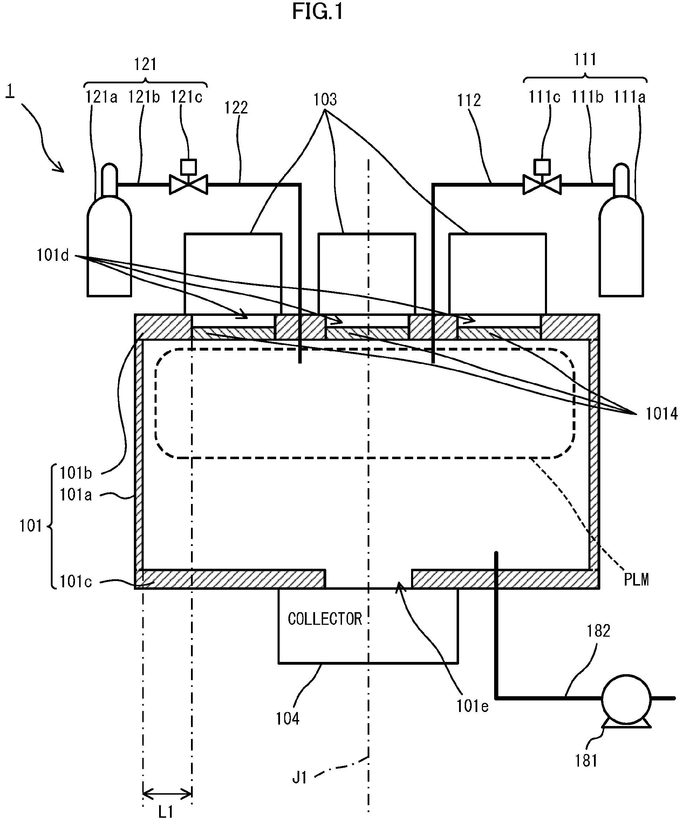

As illustrated in FIG. 1, an acetylene production apparatus 1 according to the present embodiment includes a chamber 101, a first raw material gas supplier 111, a second raw material gas supplier 121, and a collector 104 that collects acetylene gas generated in the chamber 101. Further, the acetylene production apparatus 1 includes three plasma generators 103 that generate plasma PLM in the chamber 101 by introducing an electromagnetic wave such as an RF wave, a microwave, and a millimeter wave from electromagnetic wave introduction portions 101d into the chamber 101 in a state where a first raw material gas and a second raw material gas are supplied in the chamber 101.

The chamber 101 has a cylindrical shape, and has a shape with both end portions blocked in a cylinder axis direction. An upper wall 101b of the chamber 101 is provided with the electromagnetic wave introduction portion 101d that penetrates the upper wall 101b in a thickness direction of the upper wall 101b and is a through hole for introducing the electromagnetic wave into the chamber 101 along a cylinder axis J1 of the chamber 101. A window 1014 constituted from quartz glass and the other insulating material and constituted from a material passing the electromagnetic wave is disposed inside the electromagnetic wave introduction portion 101d. Further, a lower wall 101c of the chamber 101 has a through hole 101e that penetrates the lower wall 101c in a thickness direction of the lower wall 101c and is used for an inflow of gas containing acetylene present in the chamber 101 into the collector 104.

Further, a vacuum pump 181 is connected to the chamber 101 via an exhaust pipe 182 that communicates with the inside of the chamber 101 at one end portion. The vacuum pump 181 can adopt, for example, a dry pump, and may be directly connected to the collector 104. By the vacuum pump 181 operating, gas containing the raw material gas in the chamber 101 is discharged through the exhaust pipe 182, and pressure in the chamber 101 is controlled.

The first raw material gas supplier 111 supplies the first raw material gas containing H₂ into the chamber 101. The first raw material gas 111 includes a gas storage 111a that stores the first raw material gas, a supply pipe 111b to which the first raw material gas is supplied from the gas storage 111a, and a flow rate adjustment valve 111c for adjusting a flow rate of hydrogen gas flowing through the supply pipe 111b. The hydrogen gas supplied to the supply pipe 111b is introduced into the chamber 101 via the supply pipe 112 communicating with the inside of the chamber 101 at one end portion.

The second raw material gas supplier 121 supplies the second raw material gas containing CH₄ being a group of acetylene into the chamber 101. The second raw material gas supplier 121 includes a gas storage 121a that stores the second raw material gas, a supply pipe 121b to which the second raw material gas is supplied from the gas storage 121a, and a flow rate adjustment valve 121c for adjusting a flow rate of the second raw material gas flowing through the supply pipe 121b.

The second raw material gas supplied to the supply pipe 121b is introduced into the chamber 101 via the supply pipe 122 communicating with the inside of the chamber 101 at one end portion. Herein, the first raw material gas and the second raw material gas may each include an inert auxiliary gas such as Ar and N₂ for adjusting an electron temperature and accelerating an acetylene generation reaction described below. Further, the first raw material gas and the second raw material gas can also be mixed in 112 and 122. The inert auxiliary gas such as Ar and N₂ functions like a reactive hydrogen atom radical described below.

The three plasma generators 103 each generate the plasma PLM in the chamber 101 by applying the electromagnetic wave to gas containing CH₄ and hydrogen supplied in the chamber 101. The plasma generator 103 introduces the electromagnetic wave into the chamber 101 through the window 1014 provided in the electromagnetic wave introduction portion 101d of the chamber 101. In this way, the plasma PLM is generated under the upper wall 101b provided with the window 1014 in the chamber 101.

A frequency of the electromagnetic wave output from the plasma generator 103 is not particularly limited, and can be set to, for example, a frequency from 13.56 MHz, 100 MHz, or 860 MHz to 2,450 MHz or more. Further, in the plasma generator 103, a flow direction of gas including CH₄ and hydrogen supplied in the chamber 101, and an electromagnetic wave emission port and an antenna that emits the electromagnetic wave are disposed substantially in parallel, and the electromagnetic wave propagating through them is applied to the gas.

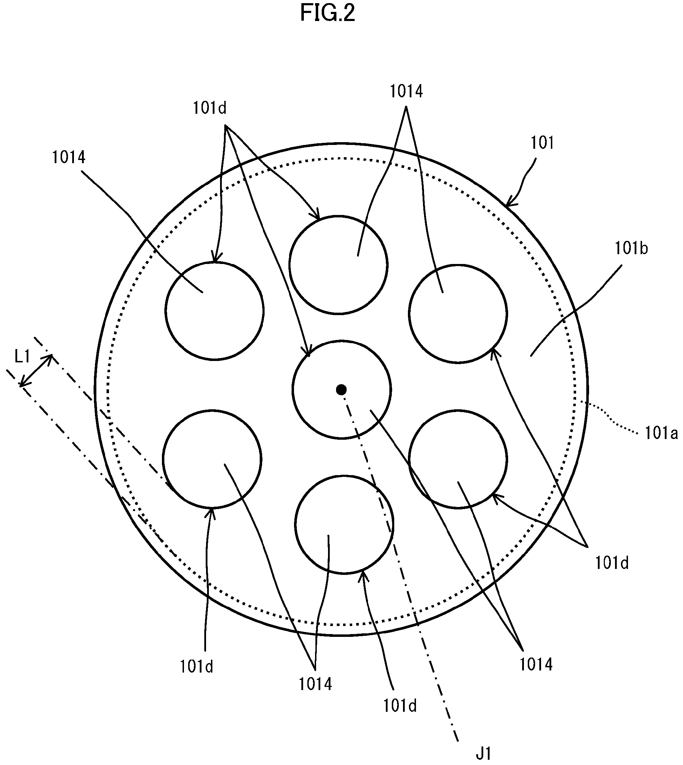

As illustrated in FIG. 2, in the acetylene production apparatus 1 according to the present embodiment, a shortest distance L1 between the electromagnetic wave introduction portion 101d and the inside of a side wall 101a of the chamber 101 in a direction orthogonal to the cylinder axis J1 direction of the chamber 101 is set longer than a vibration amplitude of an electron in the plasma PLM. The vibration amplitude of the electron can be set to a length considered as a Debye length of the electron in the plasma PLM.

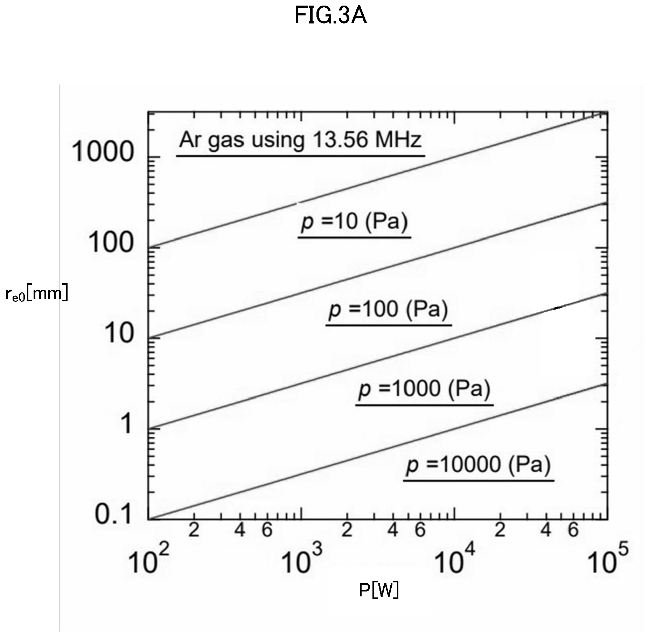

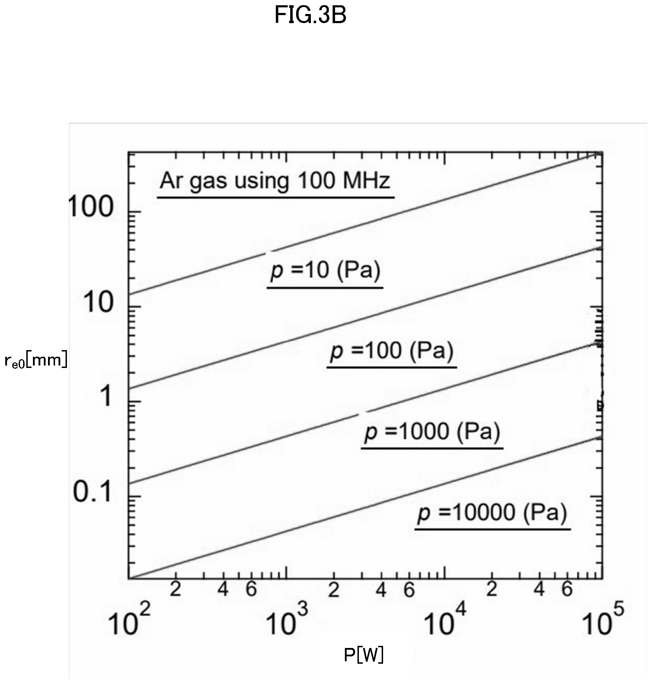

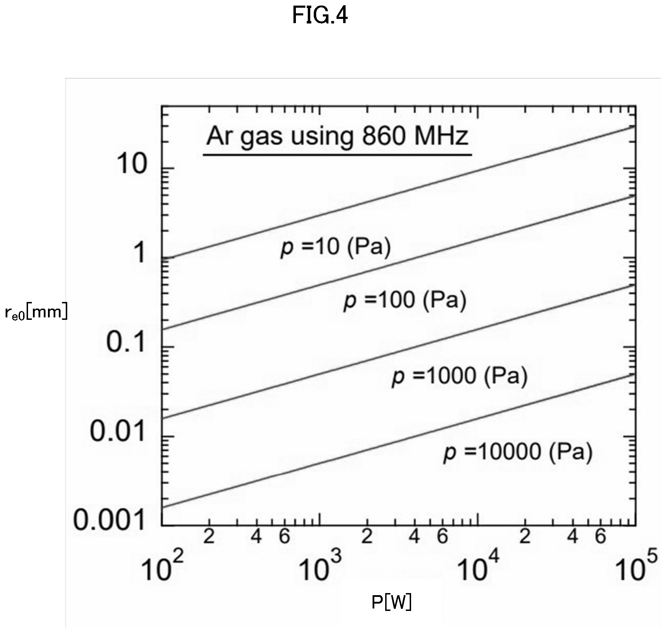

Further, as illustrated in FIGS. 3A, 3B, and 4, the vibration amplitude of the electron depends on power of the electromagnetic wave introduced into the chamber 101 and atmospheric pressure in the chamber 101. For example, when a frequency of the electromagnetic wave is 900 MHz or 13.56 MHz, electric power of the electromagnetic wave is 350 W, and atmospheric pressure in the chamber 101 is 1 kPa, the vibration amplitude of the electron is about 2 mm as illustrated in FIG. 3A. Further, for example, when a frequency of the electromagnetic wave is 100 MHz, electric power of the electromagnetic wave is 350 W, and atmospheric pressure in the chamber 101 is 1 kPa, the vibration amplitude of the electron is about 0.25 mm as illustrated in FIG. 3B.

Furthermore, for example, when a frequency of the electromagnetic wave is 860 MHz, electric power of the electromagnetic wave is 350 W, and atmospheric pressure in the chamber 101 is 1 kPa, the vibration amplitude of the electron is about 0.03 mm as illustrated in FIG. 4. In this way, the vibration amplitude of the electron is longer with a lower frequency of the electromagnetic wave. Then, in order to stably generate the plasma PLM in the chamber 101, the shortest distance L1 between the electromagnetic wave introduction portion 101d and the inside of the side wall 101a of the chamber 101 is preferably sufficiently greater than the vibration amplitude of the electron.

For example, it is assumed that atmospheric pressure in the chamber 101 is 1 kPa, a frequency of the electromagnetic wave is 13.56 MHz, and power of the electromagnetic wave is 200 W to 400 W. In this case, it is clear by referring to FIG. 3A that a vibration amplitude length of the electron is about 0.02 mm to 0.03 mm. In this case, the shortest distance L1 between the electromagnetic wave introduction portion 101d and the inside of the side wall 101a of the chamber 101 is preferably set longer than at least 0.03 mm.

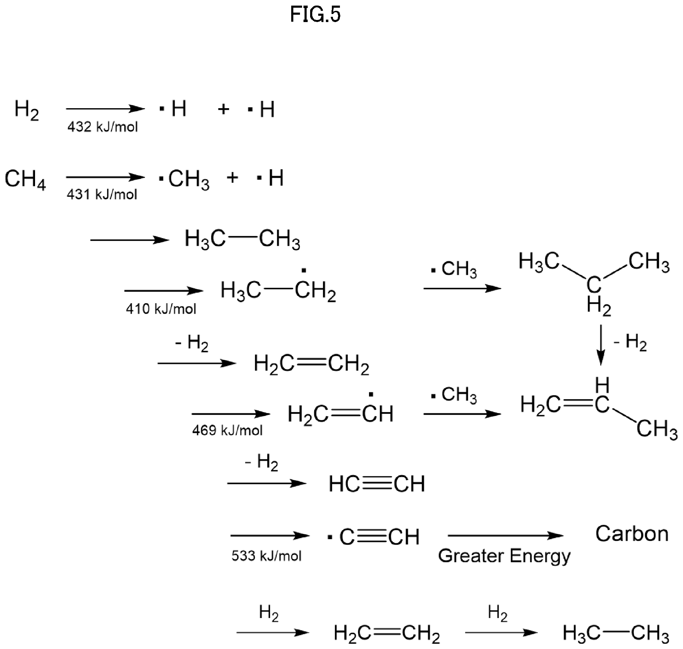

Further, in the plasma PLM, acetylene is conceivably generated by occurrence of a dimerization reaction as illustrated in FIG. 5. Specifically, a series of reactions below conceivably occurs. Note that "·H" illustrated in FIG. 5 represents a reactive hydrogen atom radical. An ·H-CH₃ radical is generated by a collision of an electron with H₂ and CH₄ present in the chamber 101. Then, CH₃ radicals are coupled to each other, and C₂H₆ is generated. Then, a C₂H₅ radical is generated by a collision of ·H and an electron with C₂H₆.

Herein, a part of the C₂H₅ radical is coupled to a CH₃ radical and C₃H₈ is generated, and C₃H₆ is generated by oxidation action by ·H on generated C₃H₈. Further, C₂H₄ is generated by the oxidation action by ·H on the C₂H₅ radical. Then, a C₂H₃ radical is generated by a collision of ·H and an electron with C₂H₄. Herein, a part of the C₂H₃ radical is coupled to the CH₃ radical and C₃H₆ is generated. Furthermore, C₂H₂ (acetylene) is generated by the oxidation action by ·H on the C₂H₃ radical. In other words, when the CH₃ radical is generated from CH₄ by the plasma PLM in the chamber 101, C₂H₆ is first generated by coupling between the CH₃ radicals.

Subsequently, a mechanism in which H₂ is successively left from C₂H₆ by the oxidation action by ·H in the plasma PLM, and C₂H₄ and C₂H₂ are generated conceivably occurs. Note that a C₂H radical is generated by a collision of ·H and an electron with C₂H₂, and carbon is generated by the oxidation action by ·H on the generated C₂H radical. Further, C₂H₄ is generated by reduction action by H₂ on C₂H₂, and, furthermore, C₂H₆ is generated by reduction action by H₂ on C₂H₄.

Further, in the acetylene production apparatus 1 according to the present embodiment, after supply of the first raw material gas containing H₂ from the first raw material gas supplier 111 into the chamber 101 starts, and after a lapse of only a preset period of time, supply of the second raw material gas containing CH₄ from the second raw material gas supplier 121 into the chamber 101 starts.

In other words, first, a first raw material gas supply step of supplying the first raw material gas containing H₂ into the chamber 101 is performed, and then a first plasma processing step of generating the plasma PLM in the chamber 101 in a state where the first raw material gas is supplied in the chamber 101 is performed, and, subsequently, a second raw material gas supply step of supplying the second raw material gas containing CH₄ into the chamber 101 is performed.

Then, a second plasma processing step of generating the plasma PLM in the chamber 101 in a state where the first raw material gas and the second raw material gas are supplied in the chamber 101 is performed. In such a manner, a state rich in ·H is achieved in the chamber 101. In this way, the CH₃ radical in FIG. 5 described above is generated. Subsequently, C₂H₆ is generated by the dimerization reaction.

From this point, C₂H₂ is generated by making abstraction of H₂ faster than another reaction with low energy. As compared with a reaction in which C₃H₈ is generated by coupling with the CH₃ radical, an abstraction reaction of H₂ in which C₃H₆ is generated by ·H is set to be dominant. Further, with regard to the C₂H₃ radical, as compared with a reaction in which C₃H₆ is generated by coupling with the CH₃ radical, a reaction in which C₂H₂ is generated by ·H is set to be dominant.

As described above, in the acetylene production apparatus 1 according to the present embodiment, the shortest distance L1 between the electromagnetic wave introduction portion 101d and the inside of the side wall 101a of the chamber 101 in the direction orthogonal to the cylinder axis J1 direction of the chamber 101 is set longer than a vibration amplitude of an electron in the plasma PLM. In this way, the electron in the plasma PLM reaching the side wall 101a of the chamber 101 and escaping into the side wall 101a of the chamber 101 can be suppressed, and thus the plasma PLM can be stably generated in the chamber 101. Therefore, a C₂H₂ generation reaction caused by a collision of the electron in the plasma PLM with CH₃ contained in the first raw material gas is accelerated, and thus a yield of C₂H₂ can be increased.

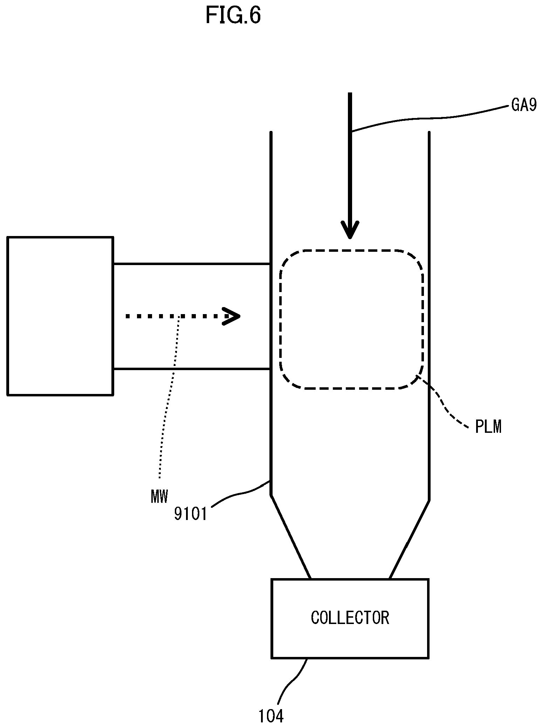

An acetylene production apparatus like, for example, an acetylene production apparatus according to Comparative Example as illustrated in FIG. 6 including a pipe body 9101 constituted from quartz glass, and a plasma generator that generates the plasma PLM by applying a microwave MW of, for example, 915 MHz to a mixed gas GA9 of CH₄, H₂, and N₂ from the side orthogonal to a cylinder axis direction of the pipe body 9101 in a state where the mixed gas GA9 flows toward the collector 104 in the pipe body 9101 is provided. Herein, the microwave MW propagating in a direction orthogonal to the flow direction of the mixed gas GA9 is applied to the mixed gas GA9.

However, the acetylene production apparatus needs to adopt a relatively thin pipe tube having an inside diameter of about 45 mm as the pipe body 9101. Thus, producing a great amount of acetylene with one pipe body 9101 is difficult. Further, since electric power of the microwave MW applied toward the pipe body 9101 is about 19 kW and relatively great, power consumption becomes massive when, for example, a plurality of acetylene production apparatuses is to produce a great amount of acetylene.

In contrast, since the acetylene production apparatus 1 according to the present embodiment has the configuration including the chamber 101 that can be increased in size and a relatively small number of the plasma generators 103, a great amount of acetylene can be produced with relatively small power consumption. In fact, when an inside diameter of the chamber 101 is set to about 550 mm, acetylene can be generated only by introducing a microwave with electric power of about 0.3 kW into the chamber 101. Therefore, an improvement in acetylene production efficiency and power conservation can be achieved. Further, a yield of C₂H₂ can be increased by increasing the size of the chamber 101.

Further, as illustrated in FIG. 3A, when atmospheric pressure in the chamber 101 is 1 kPa, a frequency of the electromagnetic wave is 900 MHz, and power of the electromagnetic wave is 200 W to 400 W, a vibration amplitude length of an electron present in the chamber 101 is about 0.02 mm to 0.03 mm. In contrast, with the configuration of the acetylene production apparatus according to the present embodiment, the shortest distance L1 between the electromagnetic wave introduction portion 101d and the inside of the side wall 101a of the chamber 101 can be set longer than 0.03 mm.

Thus, even when power of the electromagnetic wave introduced into the chamber 101 is 200 W to 400 W, the electron in the plasma PLM reaching the side wall 101a of the chamber 101 and escaping into the side wall 101a of the chamber 101 can be suppressed, and thus the plasma PLM can be stably generated in the chamber 101.

Herein, a result of performing a component analysis in gas collected by the collector 104 in each case where atmospheric pressure in the chamber 101 was set to 1 kPa, a frequency of the electromagnetic wave introduced into the chamber 101 was set to 900 MHz, and power of the electromagnetic wave was set to 200 W, 300 W, and 400 W is described. The component analysis in the gas was performed by gas chromatography mass spectrometry.

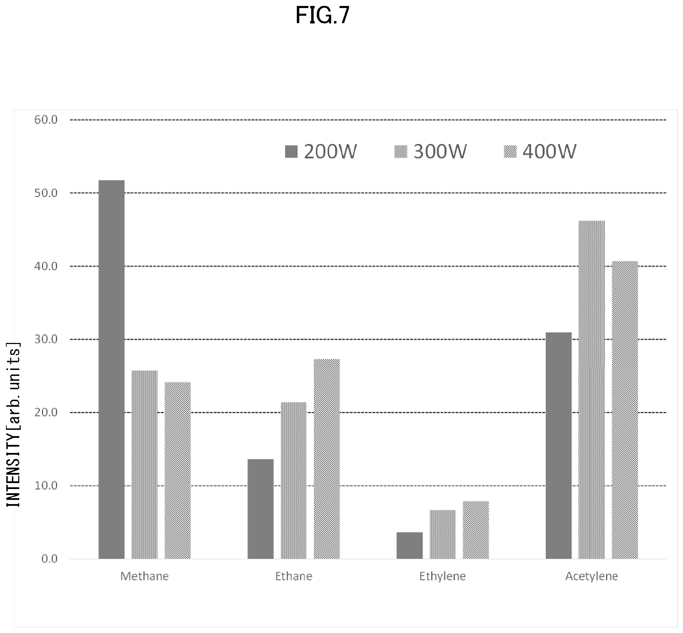

At this time, the electromagnetic wave was introduced from only one electromagnetic wave introduction portion 101d located in a central portion of the upper wall 101b of the chamber 101 in FIG. 2. As illustrated in FIG. 7, it could be confirmed that acetylene was contained in the gas collected in all of the cases where the power of the electromagnetic wave was 200 W, 300 W, and 400 W.

From this result, it is clear that the acetylene production apparatus according to the present embodiment can generate acetylene even when the power of the electromagnetic wave introduced into the chamber 101 is 200 W to 400 W that is relatively smaller than that according to Comparative Example. In this way, the acetylene production apparatus according to the present embodiment can achieve power conservation in acetylene production as compared with Comparative Example.

Further, in the acetylene production apparatus 1 according to the present embodiment, after supply of the first raw material gas containing H₂ from the first raw material gas supplier 111 into the chamber 101 starts, and after a lapse of only a preset period of time, supply of the second raw material gas containing CH₄ from the second raw material gas supplier 121 into the chamber 101 starts. In this way, abstraction of H₂ by ·H from C₂H₆ can be accelerated, and thus a yield of C₂H₂ can be increased.

### Embodiment 2

An acetylene production apparatus according to the present embodiment includes: a chamber having a cylindrical shape; a first raw material gas supplier that supplies a first raw material gas containing hydrogen into the chamber; a second raw material gas supplier that supplies a second raw material gas containing CH₄ into the chamber; and at least one plasma generator that includes a coil disposed inside the chamber or outside the chamber and an alternating current source for applying an alternating current to the coil, and generates plasma in the chamber by applying an alternating current to the coil in a state where the first raw material gas and the second raw material gas are supplied in the chamber. Then, a shortest distance between the coil and the inside of a side wall of the chamber in a direction orthogonal to a cylinder axis direction of the chamber is set longer than a vibration amplitude of an electron in the plasma.

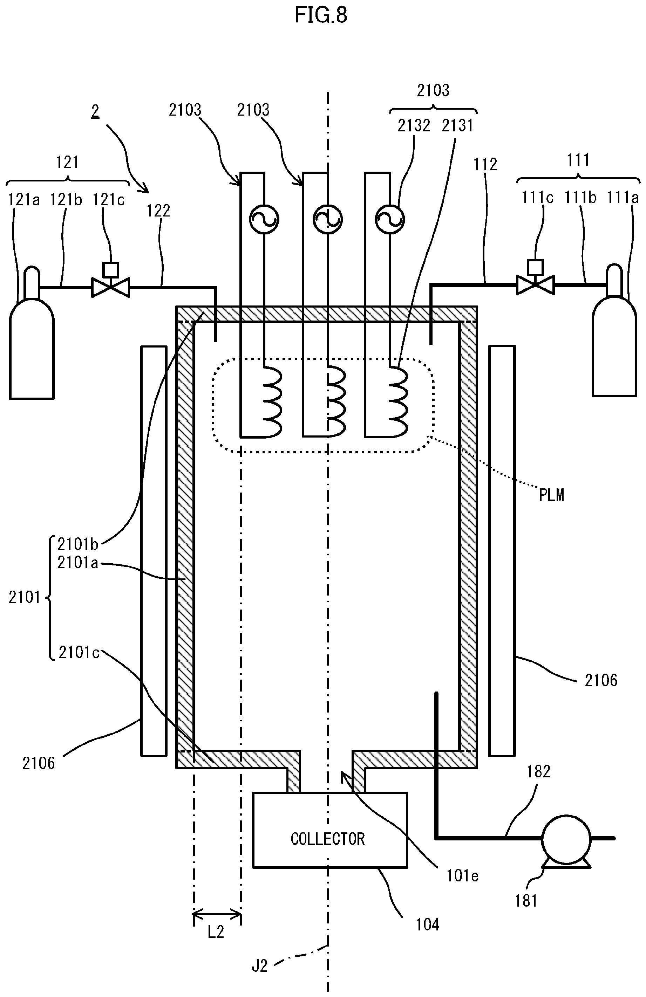

As illustrated in FIG. 8, an acetylene production apparatus 2 according to the present embodiment includes a chamber 2101, a first raw material gas supplier 111, a second raw material gas supplier 121, a collector 104, and three plasma generators 2103. Herein, the first raw material gas and the second raw material gas can also be mixed in 112 and 122. Note that, in FIG. 8, a configuration similar to that in Embodiment 1 has the same reference sign. The chamber 2101 has a cylindrical shape, and has a shape with both end portions blocked in a cylinder axis direction.

The plasma generators 2103 are attached to an upper wall 2101b of the chamber 2101. A lower wall 2101c of the chamber 2101 has a through hole 101e for an inflow of gas containing acetylene present in the chamber 2101 into the collector 104. Furthermore, the acetylene production apparatus 2 includes a cusped magnetic field generation unit 2106 that is disposed in such a way as to surround the chamber 2101 in a direction orthogonal to a cylinder axis J2 direction of the chamber 2101, and generates a cusped magnetic field in the chamber 2101.

In this way, in an acetylene generation reaction in the present embodiment, by providing the cusped magnetic field generation portion 2106 at a place where plasma PLM is generated in the chamber 2101, an electron loss in a wall of the chamber 2101 is suppressed, and density of ·H and plasma can be maintained, and thus energy supplied for newly generating an electromagnetic wave in the chamber 2101 in order to maintain the acetylene generation reaction can be reduced. Thus, energy conservation in acetylene generation can be achieved.

The three plasma generators 2103 each include a coil 2131 disposed in the chamber 2101, and an alternating current source 2132 that applies an alternating current to the coil 2131. Herein, for example, the coil 2131 may be constituted from a hollow pipe and cooled by a coolant flowing inside. Further, the coil 2131 may be provided with a protective film made of a ceramics material having heat resistance on an outer wall of the coil 2131.

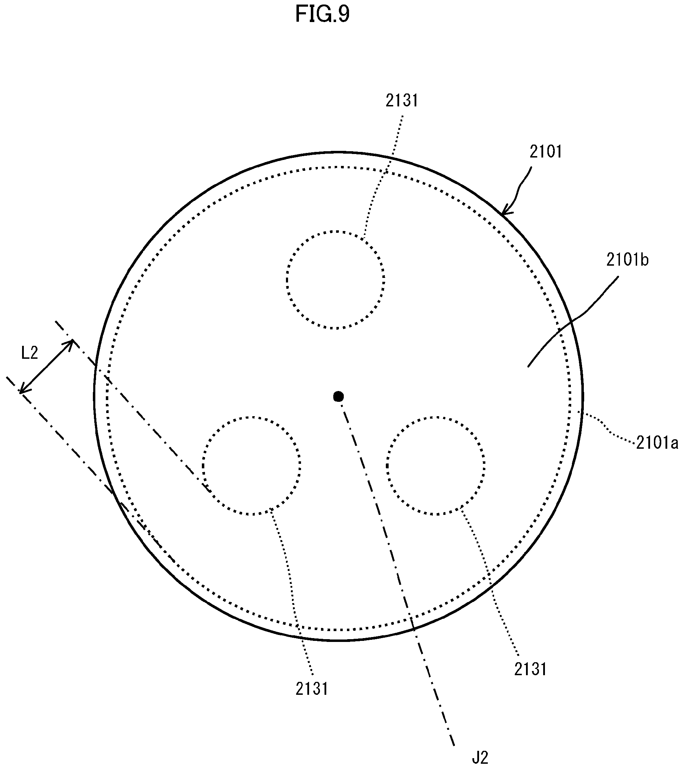

Then, the alternating current source 2132 generates the plasma PLM in the chamber 2101 by applying the alternating current having a relatively high frequency to the coil 2131 in a state where the first raw material gas and the second raw material gas are supplied in the chamber 2101. Further, as illustrated in FIG. 9, a shortest distance L2 between the coil 2131 and the inside of a side wall 2101a of the chamber 2101 in the direction orthogonal to the cylinder axis J2 direction of the chamber 2101 is set longer than a vibration amplitude of an electron in the plasma PLM.

According to the present configuration, a flow direction of gas containing CH₄ and hydrogen supplied in the chamber 2101, a winding axis direction of the coil 2131 of the plasma generator 2103, and a flow direction of a line of magnetic force of a magnetic field generated by the cusped magnetic field generation portion 2106 are substantially parallel to one another. In this way, the electromagnetic wave radiated from the plasma generator 2103 is efficiently applied to the gas containing CH₄ and hydrogen, an electron loss in a surrounding wall of the chamber 2101 is effectively suppressed, and density of ·H and plasma can be maintained.

Note that, also in the acetylene production apparatus 2 according to the present embodiment, after supply of the first raw material gas containing H₂ from the first raw material gas supplier 111 into the chamber 2101 starts, and after a lapse of only a preset period of time, supply of the second raw material gas containing CH₄ from the second raw material gas supplier 121 into the chamber 2101 may start.

Further, in the acetylene production apparatus 2 according to the present embodiment, a permanent magnet constituting the cusped magnetic field generation portion 2106 may be disposed in an inner wall portion of the chamber 2101, the outside the chamber 2101, or both in the inner wall portion and the outside of the chamber 2101. Note that, in the permanent magnet disposed in the inner wall portion of the chamber 2101, a portion of the permanent magnet exposed to plasma is covered with a protective film constituted from an insulator material such as glass.

In the acetylene production apparatus 2 according to the present embodiment, the chamber 2101 can be increased in size, and thus the permanent magnet described above can be disposed in not only an outer wall of the chamber 2101 but also an inner portion of the side wall. Further, when the acetylene production apparatus is scaled up by increasing the size of the chamber 2101, the permanent magnet constituting the cusped magnetic field generation portion 2106 is easily installed in the inner wall portion.

As described above, in the acetylene production apparatus 2 according to the present embodiment, the shortest distance L2 between the coil 2131 of the plasma generator 2103 and the inside of the side wall 2101a of the chamber 2101 in the direction orthogonal to the cylinder axis J2 direction of the chamber 2101 is set longer than a vibration amplitude of an electron in the plasma PLM.

In this way, the electron in the plasma PLM reaching the side wall 2101a of the chamber 2101 and escaping into the side wall 2101a of the chamber 2101 can be suppressed, and thus the plasma PLM can be stably generated in the chamber 2101. Therefore, a C₂H₂ generation reaction caused by a collision of the electron in the plasma PLM with CH₄ contained in the first raw material gas is accelerated, and thus a yield of C₂H₂ can be increased.

Further, the acetylene production apparatus 2 according to the present embodiment includes the cusped magnetic field generation portion 2106 that generates a cusped magnetic field in the chamber 2101. In this way, the plasma PLM can be stably generated in the chamber 2101.

Furthermore, the acetylene production apparatus 2 according to the present embodiment can increase proximity of even an electromagnetic wave (an RF wave, a microwave, and a millimeter wave) having various wavelengths with so-called low power to plasma by generating a cusped magnetic field that suppresses an electron loss, and can maintain the plasma in a high density state. In particular, an advantageous point is that a plasma generator that is relatively easy to handle, is inexpensive, and generates an electromagnetic wave at 13.56 MHz (RF band) can be adopted as the plasma generator 2103. Further, it can be expected from the acetylene production apparatus 2 according to the present embodiment that the dimerization reaction described above is further efficiently caused.

Although the embodiments of the present invention have been described above, the present invention is not limited to the configuration of the embodiments described above. For example, in Embodiment 1, four or more plasma generators 103 may be provided, or two or less plasma generator 103 may be provided. Further, in Embodiment 2, four or more plasma generators 2103 may be provided, or two or less plasma generator 2103 may be provided.

Further, in the embodiments, a value of an electron temperature in the plasma may be maintained to a value appropriate to maintain generation of ·H by setting intermittent supply of an electromagnetic wave, that is, controlling supply of an electromagnetic wave by an appropriate duty ratio. When a temperature in the chamber 101 increases, efficiency of generating C₂H₂ decreases in FIG. 5.

Thus, when a gas temperature and an electron temperature in the chamber 101 increase, a reaction in which C₂H₂ is generated in FIG. 5 smoothly proceeds by intermittently supplying the electromagnetic wave output from the plasma generator 103 and adjusting the gas temperature and the electron temperature in the chamber 101 to an appropriate temperature, and a decrease in the efficiency of generating C₂H₂ can be prevented.

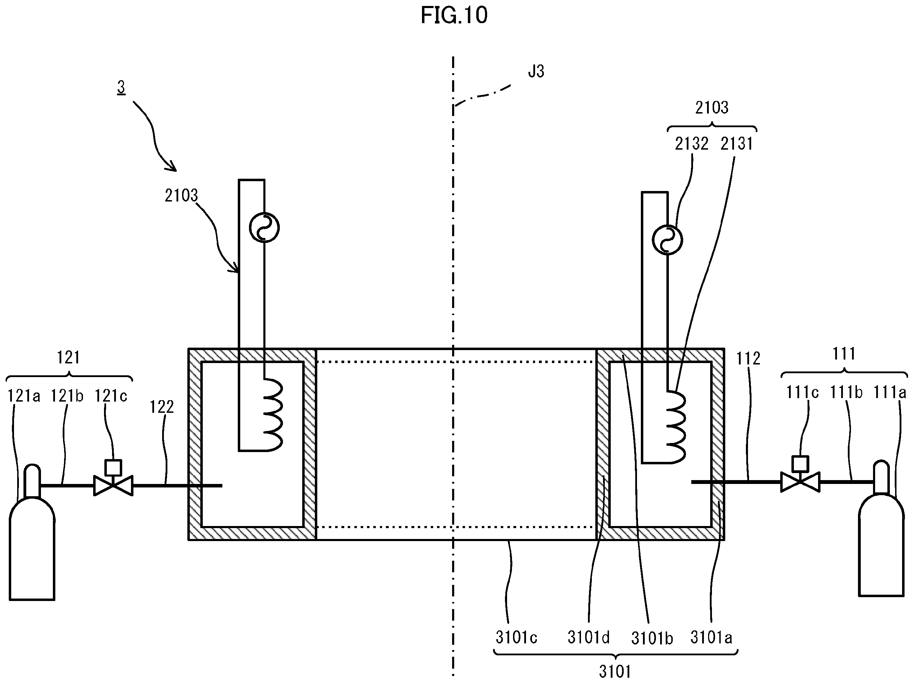

In Embodiment 2, for example, as illustrated in FIG. 10, a chamber 3101 may be hollow having an annular shape. Note that, in FIG. 10, a configuration similar to that in Embodiment 1 has the same reference sign. The chamber 3101 includes an outer side wall 3101a having a cylindrical shape, an inner side wall 3101d having a cylindrical shape and having an outside diameter shorter than an inside diameter of the outer side wall, and an upper wall 3101b and a lower wall 3101c that block both end portions in an annular axis J3 direction in a region between the outer side wall 3101a and the inner side wall 3101d. A plasma generator 2103 is attached to the outside of the upper wall 3101b of the chamber 3101. The lower wall 3101c of the chamber 3101 has a through hole (not illustrated) for an inflow of gas containing acetylene present in the chamber 3101 into a collector.

The plasma generators 2103 includes a coil 2131 disposed in such a way that a winding axis coincides with the annular axis J3 outside the chamber 2101 in the annular axis J3 direction of the chamber 3101, and an alternating current source 2132 that applies an alternating current to the coil 2131. Then, the alternating current source 2132 generates the plasma in the chamber 2101 by applying the alternating current having a relatively high frequency to the coil 2131 in a state where the first raw material gas and the second raw material gas are supplied in the chamber 3101.

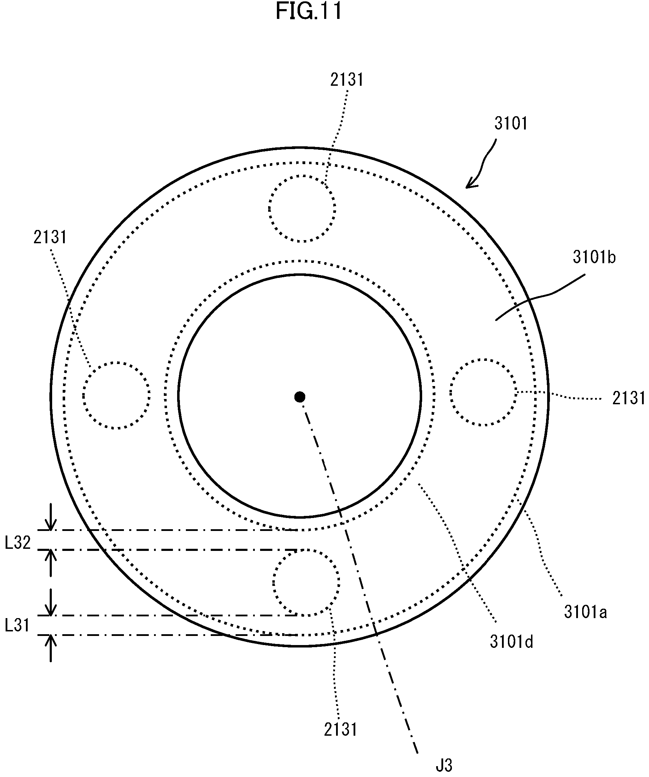

Further, as illustrated in FIG. 11, a shortest distance L31 between the coil 2131 and the inside of the outer side wall 3101a of the chamber 3101 and a shortest distance L32 between the coil 2131 and the inside of the inner side wall 3101b of the chamber 3101 in a direction orthogonal to the annular axis J3 direction of the chamber 3101 are each set longer than a vibration amplitude of an electron in the plasma PLM.

According to the present configuration, the shortest distances L31 and L32 between the coil 2131 of the plasma generator 2103 and the inside of the outer side wall 3101a of the chamber 3101 and between the coil 2131 and the inside of the inner side wall 3101d in the direction orthogonal to the annular axis J3 direction of the chamber 3101 are set longer than a vibration amplitude of the electron in the plasma PLM. In this way, the electron in the plasma PLM reaching the outer side wall 3101a or the inner side wall 3101d of the chamber 3101 and escaping into the outer side wall 3101a or the inner side wall 3101d of the chamber 3101 can be suppressed, and thus the plasma can be stably generated in the chamber 3101. Therefore, a C₂H₂ generation reaction caused by a collision of ·H and the electron in the plasma with CH₄ contained in the first raw material gas is accelerated, and thus a yield of C₂H₂ can be increased.

In the embodiments, the acetylene production apparatus may have a mechanism for collecting, as the first raw material gas containing H₂, H₂ after a reaction in the plasma PLM collected by the collector 104 and introducing the first raw material gas containing the collected H₂ into the chamber 101 again.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2023-174919, filed on October 10, 2023, the entire invention of which is incorporated by reference herein.

### Industrial Applicability

The present invention is suitable for production of acetylene using a raw material gas containing CH₄.

### List of Reference Signs

1, 2: Acetylene production apparatus
101, 2101, 3101: Chamber
101a, 2101a: Side wall
101b, 2101b, 3101b: Upper wall
101c, 2101c, 3101c: Lower wall
101d: Electromagnetic wave introduction portion
101e: Through hole
103, 2103: Plasma generator
104: Collector
111: First raw material gas supplier
111a, 121a: Gas storage
111b, 112, 121b, 122: Supply pipe
111c, 121c: Flow rate adjustment valve
121: Second raw material gas supplier
181: Vacuum pump
182: Exhaust pipe
1014: Window
2131: Coil
2132: Alternating current source
3101a: Outer side wall
3101d: Inner side wall
PLM: Plasma

## Claims

1. An acetylene production apparatus comprising:
a chamber that has a cylindrical shape, and is provided with, in at least a part of a wall blocking one end portion in a cylinder axis direction, an electromagnetic wave introduction portion for introducing an electromagnetic wave along the cylinder axis direction;
a first raw material gas supplier that supplies a first raw material gas containing H₂ into the chamber;
a second raw material gas supplier that supplies a second raw material gas containing CH₄ into the chamber; and
at least one plasma generator that generates plasma in the chamber by introducing an electromagnetic wave from the electromagnetic wave introduction portion into the chamber in a state where at least one of the first raw material gas and the second raw material gas is supplied in the chamber,
wherein a shortest distance between the electromagnetic wave introduction portion and the inside of a side wall of the chamber in a direction orthogonal to the cylinder axis direction of the chamber is set longer than a vibration amplitude length of an electron in the plasma.

2. An acetylene production apparatus comprising:
a chamber having a cylindrical shape;
a first raw material gas supplier that supplies a first raw material gas containing H₂ into the chamber;
a second raw material gas supplier that supplies a second raw material gas containing CH₄ into the chamber; and
at least one plasma generator that includes a coil disposed inside the chamber or outside the chamber and an alternating current source for applying an alternating current to the coil, and generates plasma in the chamber by applying an alternating current to the coil in a state where at least one of the first raw material gas and the second raw material gas is supplied in the chamber,
wherein a shortest distance between the coil and the inside of a side wall of the chamber in a direction orthogonal to a cylinder axis direction of the chamber is set longer than a vibration amplitude length of an electron in the plasma.

3. The acetylene production apparatus according to claim 1 or 2,
wherein, after supply of the first raw material gas from the first raw material gas supplier into the chamber starts, and after a lapse of only a preset period of time, supply of the second raw material gas from the second raw material gas supplier into the chamber starts.

4. The acetylene production apparatus according to claim 1 or 2,
further comprising a cusped magnetic field generation unit that is disposed in such a way as to surround the chamber in a direction orthogonal to the cylinder axis direction of the chamber, and generates a cusped magnetic field in the chamber.

5. An acetylene production apparatus comprising:
a chamber that is provided with: an outer side wall having a cylindrical shape; an inner side wall having a cylindrical shape and having an outside diameter shorter than an inside diameter of the outer side wall; and, in at least a part of a wall blocking one end portion in a cylinder axis direction of the outer side wall and the inner side wall in a region between the outer side wall and the inner side wall, an electromagnetic wave introduction portion for introducing an electromagnetic wave along the cylinder axis direction;
a first raw material gas supplier that supplies a first raw material gas containing H₂ into the chamber;
a second raw material gas supplier that supplies a second raw material gas containing CH₄ into the chamber; and
at least one plasma generator that generates plasma in the chamber by introducing an electromagnetic wave from the electromagnetic wave introduction portion into the chamber in a state where at least one of the first raw material gas and the second raw material gas is supplied in the chamber,
wherein a shortest distance between the electromagnetic wave introduction portion and the inside of the outer side wall of the chamber and a shortest distance between the electromagnetic wave introduction portion and the inside of the inner side wall in a direction orthogonal to the cylinder axis direction of the chamber are set longer than a vibration amplitude length of an electron in the plasma.

6. An acetylene production method comprising:
a first raw material gas supply step of supplying a first raw material gas containing H₂ into a chamber;
a first plasma processing step of generating ·H in advance in the chamber in a state where the first raw material gas is supplied in the chamber;
a second raw material gas supply step of supplying a second raw material gas containing CH₄ into the chamber after the first raw material gas supply step; and
a second plasma processing step of generating plasma in the chamber in a state where the first raw material gas and the second raw material gas are supplied in the chamber.
